# EUROPEAN PATENT APPLICATION

(11) **EP 0 884 043 A1**
(43) Date of publication of application: **16.12.1998**
(21) Application number: 98201677.6
(22) Date of filing: 20.05.1998
(51) Int. Cl.: A61K 7/00, A61K 9/12

(54) **Powder-based skin spray**

(30) Priority: 21.05.1997 NL 1006103
(71) Applicant: N.V. Nutricia, 2700 MA Zoetermeer (NL)
(72) Inventor: Vandamme, Patricia Liliane, 1072 Groot-Bijgaarden (BE); Coremans, Gerrit Alfons, 1750 Sint-Martens-Lennik (BE)
(74) Representative: de Bruijn, Leendert C.

(57) **Abstract**

The application relates to a spray for skin-hygiene and/or cosmetic purposes based on a spray powder, comprising:
- an essentially sealed pressurized container provided with means for the controlled delivery of the spray powder from the container;
- a spray powder that comprises at least one compound that is suitable for skin-hygiene and/or cosmetic purposes, the particle size of said compound being such that at least 10% by weight of the particles is larger than 10 µm;
- at least one propellant gas chosen from the class comprising the hydrofluoroalkane compounds;
- and, optionally, further carriers and/or additives for skin sprays known per se.

The compound that is suitable for skin-hygiene and/or cosmetic purposes is preferably chosen from the class comprising the imidazole compounds and/or allylamine compounds, in particular from miconazole, econazole or salts thereof that are acceptable for skin-hygiene and/or cosmetic purposes.

The propellant gas is preferably chosen from the class comprising the HFA propellant gases, in particular HFA 134a, HFA 152a and HFA 123a.

## Description

The present invention relates to a spray for skin-hygiene and/or cosmetic purposes based on a spray powder.

The invention relates in particular to such a skin spray comprising at least one compound suitable for skin-hygiene purposes and/or cosmetic purposes, in particular chosen from the class comprising the imidazole compounds and/or allylamine compounds.

Skin sprays based on skin-spray powders containing imidazole compounds are known. Said known sprays comprise in general a pressurized container containing the spray powder, as well as a propellant gas for delivering the powder from the container. Until recently, chlorofluorohydrocarbon compounds (CFCs) were used for this purpose. However, these have the disadvantage that they attack the ozone layer so that their use is no longer acceptable.

For that reason, hydrocarbons or mixtures thereof, such as mixtures of propane and butane have also in fact been used as an alternative propellant gas, for example in the skin spray that is marketed by Janssen-Cilag under the name DAKTAR-spray^{R}. Such hydrocarbons have, however, the disadvantage that they are flammable and therefore present a risk during the manufacture when the container is being filled and also for the end user.

A further problem in formulating powder-based sprays is that the powder phase may have the tendency to clot, in particular during storage, as a result of which the delivery of the powder from the container is disturbed or even completely impossible. As a result, it is not possible to use any known propellant gas in formulating a powder-based skin spray.

In addition to the abovementioned skin sprays, powder-based sprays are also known that have to be inhaled. A preparation for such a spray is described, for example, in EP 634 166. Said document describes an aerosol preparation for inhaling micronized particles, the micronized particles being coated with an ampholytic surfactant. To be suitable for inhalation, the micronized particles have to have a maximum particle size of 5 µm.

WO 90/11754 also describes a pharmaceutical preparation for inhalation that contains an aerosol of an antifungal agent. The particle size of the active substance in said preparation is such that at least 95% by weight of the particles have a particle size of 1 to 10 µm.

US 4352789 describes a powder-based aerosol preparation of a finely divided solid medicament that is coated with a dry coating of a perfluoro surfactant. Said preparation is preferably used as an inhalation preparation. In that case, the particle size is, according to said document, 1.5 to 2.0 µm.

As stated above, it is necessary in the case of powder sprays that have to be inhaled that the particles are smaller than 10 µm or even smaller than 5 µm. If, however, a powder spray is used for cosmetic and/or skin-hygiene purposes, such a particle size presents various disadvantages.

During the spraying-on of powder sprays for external application, aerosol formation ("cloud formation") generally occurs. If small particles are used, there is the risk that the particles remain suspended in the air for a long time and can therefore be inhaled.

In addition, it is advantageous if the spray is visible on the skin surface so that the user can determine visually whether the powder has been applied in the correct way. This is scarcely possible in the case of a small particle size.

Moreover, another disadvantage of small particles is that they have a stronger tendency to start to clot ("caking") than relatively large particles. As a result, blockage of the nozzle may occur. In the case of an inhalation spray, the addition of a surfactant is therefore generally necessary.

The object of the present invention is to provide a skin spray based on a compound that is suitable for skin-hygiene and/or cosmetic purposes and that does not have said disadvantages and, at the same time, provides a product acceptable to the consumer and, in particular, gives an undisturbed, controlled delivery of the powder from the container.

The invention therefore relates to a spray for skin-hygiene and/or cosmetic purposes based on a spray powder comprising:
- an essentially sealed pressurized container provided with means for the controlled delivery of the spray powder from the container;
- a spray powder that comprises at least one compound that is suitable for skin-hygiene and/or cosmetic purposes, the particle size of said compound being such that at least 10% by weight of the particles is larger than 10 µm;
- at least one propellant gas chosen from the class comprising the hydrofluoroalkane compounds;
- and, optionally, further carriers and/or additives for skin sprays known per se.

A spray of this type offers the advantage that the chance of inhaling the active substance is small. Moreover, caking of the particles is avoided and the particles can be shaken up better, as a result of which the amount of active substance sprayed onto the body part the first time will be approximately the same as during later use.

The use of the propellant according to the invention has, in addition to the elimination of the abovementioned disadvantages, the further advantage that agglomeration and/or coagulation of the (polar) solid particles of the powder phase in the (nonpolar) liquid phase is prevented.

The compound that is suitable for skin-hygiene and/or cosmetic purposes may be any compound that has a favourable action (effect) on the skin, in particular with respect to avoiding and combating infections, such as bacterial infections, fungal infections, viral infections or other forms of inflammations and also other disorders that result in an attack on or deformation/discoloration of the skin.

In this connection, the spray can be formulated and/or used to combat or prevent the disorder itself, as well as to combat or prevent the symptoms and discomfort accompanying it, such as the protection of the skin against itching, scales and small splits, discoloration (rubescence), and also for other skin-hygiene and/or cosmetic purposes, depending on the final composition.

The compound may furthermore also have a purely cosmetic action or a purely hygienic/cleansing action, and compounds suitable for this purpose will be clear to the person skilled in the art. Compounds having a combined hygienic and cosmetic action are, however, preferred.

The compounds used must be suitable for effective use on the skin, whether topical or not. Furthermore, the compounds must be suitable to be formulated into a (skin) spray powder. For this purpose, compounds that are liquid per se and that can be formulated with a suitable solid carrier to form a powder may be used. Preferably, however, compounds are used that are solid under the conditions of use; for compounds of this type, use as spray powder is as a rule the most suitable application form, both from the point of view of the final effectiveness of the active compound and also from the point of view of the user's comfort.

Examples of suitable compounds are:
- antiseptic agents such as chlorhexidine and polyvidone-iodine (isobetadine);
- antibacterial agents such as chloramphinecol and its derivatives, and also antibiotics such as neomycin, gentamycin and tetracycline;
- anti-inflammatory agents such as corticosteroids, for example hydrocortisone and its derivatives;
- antiviral agents such as acyclovir.

In this connection, it will be clear to the person skilled in the art that such compounds may also have a combined action against (for example) both fungal infections and bacterial infections, possibly depending on the amount used.

The compound that is suitable for skin-hygiene and/or cosmetic purposes is preferably chosen from the classes of the imidazole compounds and/or allylamine compounds. These compounds are effective, in particular, against fungal infections.

Examples of imidazole compounds that are suitable for skin-hygiene and/or cosmetic purposes are miconazole, econazole, bifonazole, clotrimazole, sulconazole, ketoconazole, butoconazole and the like; as well as the salts thereof, such as the nitrate salts, that are acceptable for skin-hygiene and/or cosmetic purposes.

Examples of allylamine compounds that are suitable for skin-hygiene and/or cosmetic purposes are nystatin as well as its salts that are acceptable for skin-hygiene and/or cosmetic purposes.

More preferably, imidazole compounds that are suitable for skin-hygiene and/or cosmetic purposes are used and more preferably miconazole, econazole or salts thereof that are suitable for skin-hygiene and/or cosmetic purposes, in particular miconazole nitrate and/or econazole nitrate are used.

The abovementioned compounds may optionally also be used in a suitable combination, optionally to obtain a combined and/or synergistic action or to obtain a broad action spectrum.

The particle size of the compound that is suitable for skin-hygiene and/or cosmetic purposes is such that at least 10% by weight of the particles are larger than 10 µm. Preferably, at least 50% by weight of the particles are larger than 10 µm. Still more preferably, at least 90% by weight of the particles are larger than 10 µm.

The propellant gas is chosen from the class of hydrofluoroalkane compounds, more particularly from propellant gases having the general designation HFA, such as HFA 123a, HFA 134a and/or HFA 152a and/or mixtures thereof. HFA 134a is most preferably used.

The spray powder will generally contain the compound acceptable for skin-hygiene and/or cosmetic purposes in combination with a suitable carrier for formulating a skin spray powder.

Furthermore, the sprays may comprise all the additives known per se for sprays and/or spray powders, in particular for use on the skin, more particularly, for skin-hygiene and/or cosmetic purposes, and these will be known to the person skilled in the art.

Examples of suitable carrier materials and/or additives are, inter alia:
- inert fillers or compounding agents such as talc, silica, zinc undecylenate;
- inert solvents, such as alcohols, ethers, triglycerides;
- scenting agents such as perfumes;
- preserving agents and/or biocides.

In those cases where rehydration of the skin or repair of the hydrolipid layer attacked by the fungus is necessary, a suitable lipid for this purpose may be added. Examples of these are triglycerides, in particular medium-chain triglycerides, or MCT oils, such as the triglycerides of capric acid and/or caprylic acid.

The spray may furthermore comprise preservatives, preferably organic zinc compounds, more particularly zinc undecylenate, in an amount of 0.01-5% by weight, preferably 0.5-1.5% by weight.

As filler/carrier, use is preferably made of a fine mineral talc, which may also ensure absorption of excess perspiration. The use of silica in the spray of the invention may ensure a uniform distribution of the powder. Triglycerides may contribute to a good skin feel.

The compound that is suitable for skin-hygiene and/or cosmetic purposes is preferably present in an amount of 0.01-10% by weight, preferably 1-5% by weight, relative to the total weight of the spray powder and depending on the compound used.

The preferred imidazole compounds, i.e. miconazole nitrate and/or econazole nitrate, can be suitably used, in particular, in an amount of 1.5-2.5% by weight, more particularly approximately 2% by weight, relative to the total weight of the spray powder. This is equivalent to approximately 20 mg of miconazole nitrate/econazole nitrate per gram of powder.

The container may be any suitable container that is resistant to the internal pressure used for the spray (i.e. a suitable pressure of 1.1-100 bar, in particular 5-20 bar), having any suitable capacity, size and/or shape. The container is preferably in the shape of a (cylindrical) spray can having a capacity of 10-1000 ml, more particularly 50-250 ml.

The container furthermore comprises means for delivering the spray powder in a controlled manner, such as a valve or another suitable, sealable nozzle. Said means preferably ensure a uniform delivery of the spray powder, i.e. delivery of a controlled unit dose or of a controlled amount per unit time.

The spray can be produced in a manner known per se, analogously to the production of known skin sprays, a hydrofluoroalkane being used, however, as propellant gas according to the invention. In this connection, the skin spray powder will be formulated in a manner known per se and introduced together with the propellant gas and the optional further ingredients into the container, which is then sealed. The spray is then immediately suitable for final use.

As is known, the final spray will, as a rule, comprise three phases, namely a solid spray-powder phase, a liquid phase that comprises the solvents used as well as any portion of the propellant gas in liquid form, and the gaseous propellant gas. In this connection, the desired additives may have been taken up or be taken up in a suitable manner in the spray-powder phase or in the liquid phase, depending on the nature of the additive.

All the ingredients used must, of course, be suitable for use in a spray. In this connection, care has to be taken, in particular, that the spray does not become clogged during use (i.e. during the delivery of the spray powder); the ingredients must be chosen in a suitable manner for this purpose on the basis of factors such as particle size and the like, as will be clear to the person skilled in the art.

As described above, the use of the propellant gas according to the invention has the further advantage in this connection that agglomeration and/or coagulation of the (polar) solid particles of the powder phase in the (nonpolar) liquid phase is prevented. It has been found that the composition of the various phases (i.e. powder phase, liquid phase and propellant), and in particular the ratios between said

phases, also plays/play an important role in this connection, in particular, a compound within the following ranges has, in particular, been found favourable:
- - solid phase:: 5-8% by weight
of which talc forms:0.01-3% by weight
- - liquid phase:: 5-8% by weight
- - propellant gas:: 80-90% by weight
totalling 100% by weight and all relative to the total content of the container, the solid phase comprising an effective amount of the compound suitable for skin-hygiene and/or cosmetic purposes.

To avoid clogging, it has further been found according to the invention that a valve having an essentially round nozzle with a diameter of 0.1-1 mm, more particularly 0.5-0.7 mm, offers great advantages, in particular in combination with the spray powder described herein.

As stated above, the spray according to the invention can be used for skin-hygiene and/or cosmetic purposes, depending on its final composition.

The spray based on the abovementioned imidazole and/or allylamine compound can be used, in particular, to protect the skin against itching, scales and small splits, to prevent or combat fungal infections, to prevent or combat discoloration (rubescence) and also for other skin-hygiene and/or cosmetic purposes, depending on the final composition. Said spray is suitable, in particular, for use on the feet, in which connection the spray may be formulated so as to absorb or combat perspiration and/or so as to remove or mask smell.

The invention relates furthermore to the use of a propellant gas from the class comprising the hydrofluoroalkane compounds in formulating a skin spray based on a skin spray powder or for dispersing/nebulizing a skin spray powder, in particular in or out of a suitable container, respectively, the skin spray powder comprising a compound suitable for skin-hygiene and/or cosmetic purposes, as described above.

The invention will now be explained by reference to the nonrestricting example below.

### Example

A hair spray was formulated on the basis of the following ingredients.
Spray powder phase: Total 7 g, composed of:
   miconazole nitrate (0.2 g), 50% by weight of the particles larger than 9.7 µm, fine mineral talc; silica; zinc undecylenate, 50% by weight of the particles larger than 60 µm.
Liquid phase: Total 5 g, composed of:
   ethanol; didecylammonium chloride (biocide); capric acid/caprylic acid triglycerides; perfume.

The spray powder phase and the liquid phase were introduced together with HFA 134a as propellant gas and dimethyl ether as further solvent into a cylindrical container having a volume of 125 ml and provided with a valve having a diameter of 0.57 mm (final internal pressure approximately 18 bar). The container was sealed and the spray was ready for use.

## Claims

1. Spray for skin-hygiene and/or cosmetic purposes based on a spray powder, comprising:
- an essentially sealed pressurized container provided with means for the controlled delivery of the spray powder from the container;
- a spray powder that comprises at least one compound that is suitable for skin-hygiene and/or cosmetic purposes, the particle size of said compound being such that at least 10% by weight of the particles is larger than 10 µm;
- at least one propellant gas chosen from the class comprising the hydrofluoroalkane compounds;
- and, optionally, further carriers and/or additives for skin sprays known per se.

2. Spray according to Claim 1, wherein the compound that is suitable for skin-hygiene and/or cosmetic purposes is chosen from the class comprising the imidazole compounds and/or allylamine compounds.

3. Spray according to Claim 1 or 2, wherein the compound that is suitable for skin-hygiene and/or cosmetic purposes is chosen from the class comprising the imidazole compounds and/or allylamine compounds, more particularly from miconazole, econazole, bifonazole, clotrimazole, sulconazole, ketoconazole, butoconazole or salts thereof that are suitable for skin-hygiene and/or cosmetic purposes.

4. Spray according to any of the preceding claims, wherein the compound that is suitable for skin-hygiene and/or cosmetic purposes is miconazole, econazole or a salt thereof that is acceptable for skin-hygiene and/or cosmetic purposes, in particular miconazole nitrate of econazole nitrate.

5. Spray according to any of the preceding claims, wherein the particle size of said compound is such that at least 50% by weight of the particles are larger than 10 µm.

6. Spray according to Claim 5, wherein at least 90% by weight of the particles are larger than 10 µm.

7. Spray according to any of the preceding claims, wherein the propellant gas is chosen from the classes comprising the HFA propellant gases, in particular HFA 134a, HFA 152a and HFA 123a.

8. Spray according to Claim 1 or 2, wherein the propellant gas is HFA 134a.

9. Spray according to any of the preceding claims, further containing zinc undecylenate in an amount of 0.01-5% by weight.

10. Spray according to any of the preceding claims, composed of a solid phase containing the spray powder, a liquid phase that contains solvents and liquid propellant gas and a gas phase that contains propellant gas, wherein the solid phase is 5 to 8% by weight, the liquid phase 5 to 8% by weight and the gas phase 80 to 90% by weight of the total weight of the ingredients of the spray.

11. Spray according to any of the preceding claims, wherein the means for delivering the spray powder comprise a valve having a nozzle with a diameter of 0.1-1 mm, more particularly 0.5-0.7 mm.

12. Use of a propellant gas from the class comprising the hydrofluoroalkane compounds in formulating a skin spray based on a skin spray powder or for dispersing/nebulizing a skin spray powder, in particular in or out of a suitable container, respectively, wherein the skin spray powder comprises a compound that is suitable for skin-hygiene and/or cosmetic purposes from the class comprising the imidazole compounds and/or allylamine compounds.
